# FASCICULE DE BREVET EUROPEEN

(11) **EP 0 414 610 B1**
(45) Date de publication et mention de la délivrance du brevet: **26.05.1993**
(21) Numéro de dépôt: 90402333.0
(22) Date de dépôt: 22.08.1990
(51) Int. Cl.: C07C 233/83, C07C 231/18, C07C 271/22, C07C 269/00, C07C 227/22, C07D 305/14

(54) **Procédé pour la préparation énantiosélective de dérivés de la phénylisosérine**
Verfahren zur enantioselektiven Herstellung von Phenylisoserin-Derivaten
Process for the enantioselective preparation of phenylisoserine derivatives

(30) Priorité: 23.08.1989 FR 8911162; 02.10.1989 FR 8912825
(43) Date de publication de la demande: 27.02.1991
(73) Titulaire: CENTRE NATIONAL DE LA RECHERCHE SCIENTIFIQUE, 75007 Paris (FR)
(72) Inventeur: Correa, Arlène Gonçalves, F-38100 Grenoble (FR); Denis, Jean-Noel, Le Pinet, F-38410 Uriage (FR); Greene, Andrew Elliot, St Martin D'Uriage, F-38410 Uriage (FR)
(74) Mandataire: Pilard, Jacques

(56) Documents cités:
- EP-A- 0 253 738
- DE-A- 2 545 818
- THE JOURNAL OF ORGANIC CHEMISTRY, vol. 51, no. 1, 10 janvier 1986, pages 46-50, American Chemical Society, US; J.-N. DENIS et al.: "An efficient, enantioselective synthesis of the taxol side chain"
- JOURNAL OF THE AMERICAN CHEMICAL SOCIETY, vol. 110, no. 17, 17 août 1988, pages 5917-5919, American Chemical Society, US; J.-N. DENIS et al.: "A highly efficient, practical approach to natural taxol"
- THE JOURNAL OF ORGANIC CHEMISTRY, vol. 55, no. 6, 16 mars 1990, pages 1957-1959, American Chemical Society, US; J.-N. DENIS et al.: "An improved synthesis of the taxol side chain and of RP 56976"

## Description

La présente invention concerne un procédé pour la préparation énantiosélective de dérivés de la phénylisosérine de formule générale : 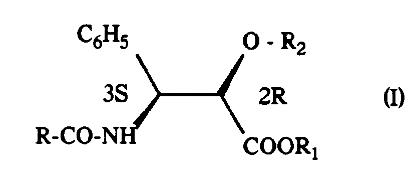
dans laquelle R représente un radical phényle ou tert.butoxy, R₁ représente un atome d'hydrogène ou un radical alkyle contenant 1 à 4 atomes de carbone et R₂ représente un atome d'hydrogène ou un groupement protecteur de la fonction hydroxy.

Dans la formule générale (I), R₂ représente plus particulièrement un atome d'hydrogène ou un radical méthoxyméthyle, éthoxy-1 éthyle, benzyloxyméthyle, (β-triméthylsilyléthoxy) méthyle, tétrahydropyrannyle ou trichloro-2,2,2 éthoxycarbonyle. De préférence, le radical R₂ est le radical éthoxy-1 éthyle.

Les produits de formule générale (I) sont utiles pour préparer les dérivés de la baccatine III et de la désacétyl-10 baccatine III de formule générale : 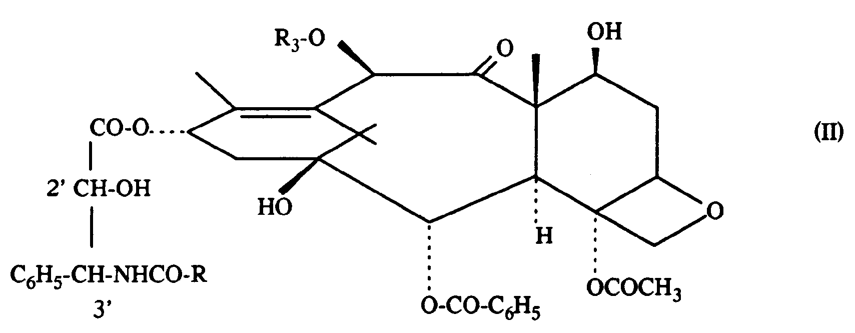
dans laquelle R₃ représente un atome d'hydrogène ou un radical acétyle et R représente un radical phényle ou un radical tert.butoxy.

Dans l'article de J-N. Denis et al., J. Org. Chem., 51, 46 (1986) est décrite la préparation du produit de formule générale (I) dans laquelle -OR₂ représente un radical hydroxy et R représente un radical phényle, par action du chlorure de benzoyle sur l'azido-3 hydroxy-2 phényl-3 propionate de méthyle suivie de la réduction et de la transposition de l'azido-3 benzoyloxy-2 phényl-3 propionate de méthyle préalablement isolé.

Les produits de formule générale (II) dans laquelle R représente un radical phényle correspondent au taxol et au désacétyl-10 taxol et les produits de formule générale (II) dans laquelle R représente un radical tert.butoxy correspondent à ceux qui sont décrits dans le brevet européen EP 253 738.

Les produits de formule générale (II), et en particulier ceux qui se présentent sous la forme 2'R, 3'S, présentent des propriétés antitumorales et antileucémiques particulièrement intéressantes.

Les produits de formule générale (II) peuvent être obtenus par action d'un produit de formule générale (I) dans laquelle R₁ représente un atome d'hydrogène sur un dérivé du taxane de formule générale : 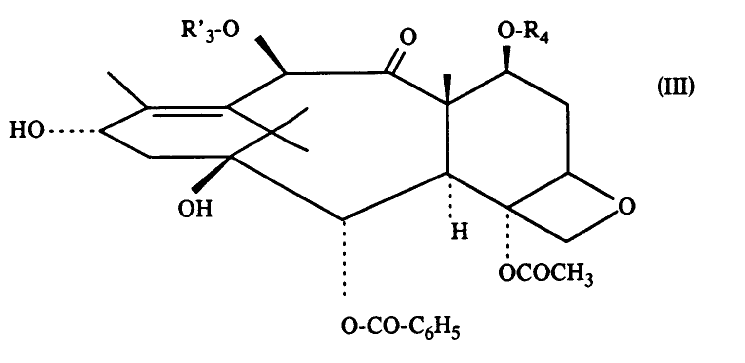
dans laquelle R'₃ représente un radical acétyle ou un groupement protecteur de la fonction hydroxy et R₄ représente un groupement protecteur de la fonction hydroxy, suivie du remplacement des groupements protecteurs R₂ et R₄ et éventuellement R₃ par un atome d'hydrogène, dans les conditions décrites par J-N. DENIS et coll., J. Amer. Chem. Soc., 110(17) 5917-5919 (1988) ou par L. Mangatal et coll., Tetrahedron, 45, 4177-4190 (1989).

Selon la présente invention, les produits de formule générale (I), dans laquelle R représente un radical phényle ou tert.butoxy, R₁ représente un radical alkyle contenant 1 à 4 atomes de carbone et R₂ représente un groupement protecteur de la fonction hydroxy, sont obtenus à partir d'un dérivé de l'oxirane de formule générale : 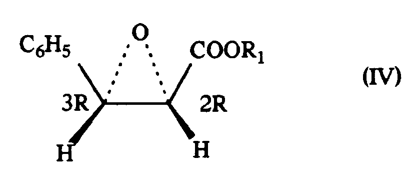
dans laquelle R₁ est défini comme précédemment, qui est traité par un azoture pour donner un produit de formule générale : 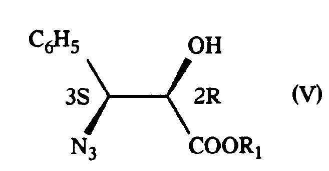
dans laquelle R₁ est défini comme précédemment, dont la fonction hydroxy est ensuite protégée par un groupement R₂ de façon à obtenir un produit de formule générale : 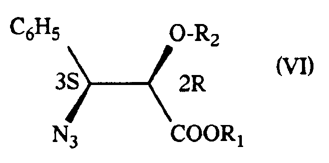
dans laquelle R₁ est défini comme précédemment et R₂ représente un groupement protecteur de la fonction hydroxy, qui est réduit en produit de formule générale : 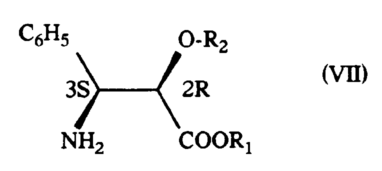
dans laquelle R₁ et R₂ sont définis comme précédemment, qui est traité par un réactif permettant d'introduire un groupement benzoyle ou tert.butoxycarbonyle pour obtenir un produit de formule générale (I) dans laquelle R₁ représente un radical alkyle contenant 1 à 4 atomes de carbone qui est ensuite saponifié pour donner un produit de formule générale (I) dans laquelle R₁ représente un atome d'hydrogène.

L'ouverture de l'oxirane de formule générale (IV) en produit de formule générale (V) peut être effectuée par action d'un azoture en opérant dans un solvant organique convenable à une température généralement comprise entre 40 et 80°C. Il est avantageux d'utiliser l'azoture de triméthylsilyle en présence de chlorure de zinc ou un azoture de métal alcalin (sodium, potassium, lithium) en milieu hydro-méthanolique en présence de formiate de méthyle.

Le produit de formule générale (VI) peut être obtenu à partir du produit de formule générale (V) dans les conditions habituelles de préparation des éthers et des acétals et plus particulièrement selon les procédés décrits par J-N. DENIS et coll., J.Org. Chem., 51, 46-50 (1986).

Le produit de formule générale (VII) peut être obtenu par réduction du produit de formule générale (VI) au moyen d'hydrogène en présence d'un catalyseur d'hydrogénation tel que le palladium sur charbon en opérant dans un alcool tel que le méthanol.

Le produit de formule générale (I) dans laquelle R₁ représente un radical alkyle contenant 1 à 4 atomes de carbone peut être obtenu par action du dicarbonate de di-tertiobutyle ou de chlorure de benzoyle sur le produit de formule générale (VII).

Généralement, on opère dans un solvant organique tel que le chlorure de méthylène en présence d'une base minérale telle que le bicarbonate de sodium ou d'une base organique telle qu'une amine tertiaire comme la triéthylamine. Il n'est pas nécessaire d'isoler le produit de formule générale (VII) pour faire réagir le dicarbonate de di-tertiobutyle ou de chlorure de benzoyle.

Le dérivé d'oxirane de formule générale (IV) peut être obtenu dans les conditions décrites par J-N. DENIS et coll. J. Org., Chem., 51 46-50 (1986).

Selon la présente invention, les produits de formule générale (I) dans laquelle R₁ représente un radical alkyle contenant 1 à 4 atomes de carbone et R₂ représente un atome d'hydrogène peuvent être obtenus par acylation-hydrogénation d'un hydroxy-azoture de formule générale (V) dans laquelle R₁ est défini comme précédemment.

L'acylation et l'hydrogénation peuvent être réalisées simultanément ou successivement sans isolement des produits intermédiaires de la réaction.

Lorsque R représente un radical phényle ou un radical tert.butoxy, la réaction peut être mise en oeuvre en utilisant l'anhydride benzoïque ou le dicarbonate de di-tert.butyle en présence d'hydrogène et d'un catalyseur d'hydrogénation tel que la palladium sur charbon en opérant dans un solvant organique inerte tel qu'un ester comme l'acétate de méthyle ou l'acétate d'éthyle à une température comprise entre 0 et 40°C, de préférence voisine de 20°C.

Lorsque R représente un radical phényle, la réaction peut être mise en oeuvre en utilisant le chlorure de benzoyle en présence d'une base organique telle que la triéthylamine et d'un agent d'activation tel que la diméthylamino-4 pyridine puis en ajoutant du méthanol et ensuite en plaçant le mélange réactionnel sous atmosphère d'hydrogène en présence d'un catalyseur d'hydrogénation tel que le palladium sur charbon en opérant dans un solvant organique inerte tel qu'un ester comme l'acétate de méthyle ou l'acétate d'éthyle à une température comprise entre 0 et 40°C et de préférence voisine de 20°C.

Le produit de formule générale (I), dans laquelle R₁ représente un radical alkyle contenant 1 à 4 atomes de carbone et R₂ représente un groupement protecteur de la fonction hydroxy, peuvent être obtenus à partir d'un produit de formule générale (I) dans laquelle R₁ représente un radical alkyle contenant 1 à 4 atomes de carbone et R₂ représente un atome d'hydrogène dans les conditions habituelles de préparation des éthers et des acétals citées précédemment.

Le produit de formule générale (I), dans laquelle R₁ représente un atome d'hydrogène peut être obtenu par saponification d'un produit de formule générale (I) dans laquelle R₁ représente un radical alkyle au moyen d'une base minérale telle qu'un hydroxyde de métal alcalin (lithium, sodium), un carbonate ou bicarbonate de métal alcalin (bicarbonate de sodium, carbonate ou bicarbonate de potassium) en milieu hydro-alcoolique tel qu'un mélange méthanol-eau à une température comprise entre 10 et 40°C, de préférence voisine de 25°C.

La présente invention concerne également la préparation du produit de formule générale (II) par condensation sur un produit de formule générale (III) dans laquelle les groupements protecteurs représentés par R'₃ et R₄ sont généralement des groupements trichloro-2,2,2 éthoxycarbonyle ou des radicaux trialkylsilyles dont chaque partie alkyle contient 1 à 3 atomes de carbone d'un dérivé de la phénylisosérine de formule générale (I) dans laquelle R₁ représente un atome d'hydrogène et R₂ représente un groupement protecteur de la fonction hydroxy, qui est obtenu en faisant réagir un azoture choisi parmi les azotures alcalins ou l'azoture de triméthylsilyle sur un dérivé de l'oxirane de formule générale (IV) dans laquelle R₁ représente un atome d'hydrogène ou un radical alcoyle contenant 1 à 4 atomes de carbone pour obtenir un produit de formule générale (V) sur lequel on fait réagir simultanément ou successivement, après avoir éventuellement protégé la fonction hydroxy, l'hydrogène et un réactif permettant d'introduire un radical benzoyle ou t.butoxycarbonyle, sans isoler les produits intermédiaires de réaction, pour obtenir, après protection de la fonction hydroxy, un produit de formule générale (I) dans laquelle R₁ est défini comme précédemment et R₂ représente un groupement protecteur de la fonction hydroxy, qui est éventuellement saponifié, suivie du remplacement des groupements protecteurs par un atome d'hydrogène.

Généralement, l'estérification est effectuée en présence d'un agent de condensation tel qu'un carbodiimide comme le dicyclohexylcarbodiimide ou un carbonate réactif comme le dipyrilyl-2 carbonate et d'un agent d'activation tel qu'une dialcoylaminopyridine comme la diméthylamino-4 pyridine en opérant dans un solvant organique aromatique tel que le benzène, le toluène, les xylènes, l'éthylbenzène, l'isopropylbenzène ou le chlorobenzène à une température comprise entre 60 et 90°C.

Il est particulièrement avantageux d'utiliser un excès molaire d'acide de formule générale (I) par rapport au dérivé du taxane de formule générale (III), l'agent de condensation étant utilisé en quantité stoechiométrique par rapport à l'acide de formule générale (I) et la diméthylamino-4 pyridine étant utilisée en quantité stoechiométrique par rapport au dérivé du taxane de formule générale (III).

Le remplacement des groupements protecteurs par un atome d'hydrogène peut être effectué au moyen de zinc en présence d'acide acétique à une température comprise entre 30 et 60°C ou par traitement au moyen d'un acide minéral ou organique tel que l'acide chlorhydrique ou l'acide acétique en solution dans un alcool aliphatique contenant 1 à 3 atomes de carbone en présence de zinc.

Les exemples suivants, donnés à titre non limitatif, montrent comment l'invention peut être mise en pratique.

### EXEMPLE 1

Dans un ballon de 25 cm3 muni d'une agitation magnétique, on introduit successivement, sous atmosphère d'argon, 530 mg de (+)-azido-3 hydroxy-2 phényl-2 propionate de méthyle (2,4 mmoles), 12 cm3 de dichlorométhane sec, 60,2 mg de p.toluènesulfonate de pyridinium (0,24 mmole) et 2,3 cm3 d'éthyl vinyl éther (24 mmoles). On agite pendant 4 heures à une température voisine de 20°C. La réaction terminée, on ajoute une goutte de pyridine puis on ajoute 50 cm3 de dichlorométhane. Le mélange réactionnel est lavé deux fois par 5 cm3 d'eau et deux fois avec 5 cm3 d'une solution aqueuse saturée de chlorure de sodium puis séché sur sulfate de sodium anhydre. Après filtration et évaporation du solvant, on obtient 706 mg d'azido-3 (éthoxy-1 éthoxy)-2 phényl-3 propionate de méthyle sous forme d'un mélange équimoléculaire des 2 épimères.

Dans un ballon de 25 cm3, muni d'une agitation magnétique, on introduit successivement, sous atmophère d'argon, 0,6 g d'azido-3(éthoxy-1 éthoxy)-2 phényl-3 propionate de méthyle (2,05 mmoles), 16,4 cm3 de méthanol, 0,714 cm3 de triéthylamine (5,12 mmoles) et 107 mg de palladium sur charbon à 10 % en poids de palladium. Le mélange obtenu est mis sous atmosphère d'hydrogène puis on l'agite vigoureusement pendant 4 heures à une température voisine de 20°C. Lorsque la réaction est terminée, le mélange réactionnel est mis sous atmosphère d'argon. Le catalyseur est séparé par filtration sur célite. Le catalyseur est lavé trois fois avec 15 cm3 de chlorure de méthylène. Les phases organiques réunies sont concentrées à sec sous pression réduite. On obtient un résidu liquide incolore.

Dans un ballon de 25 cm3, muni d'une agitation magnétique, on introduit, sous atmosphère d'argon, le résidu obtenu précédemment et 10,3 cm3 de dichlorométhane. A la solution obtenue, on ajoute 1,43 cm3 de triéthylamine (10,25 mmoles) et 536,9 mg de dicarbonate de di-t-butyle (2,46 mmoles). On agite le mélange réactionnel pendant 43 heures à une température voisine de 20°C puis on ajoute 100 cm3 d'acétate d'éthyle. Le mélange réactionnel est lavé deux fois avec 10 cm3 d'une solution aqueuse saturée de bicarbonate de sodium, 3 fois avec 5 cm3 d'eau et 1 fois avec 5 cm3 d'une solution aqueuse saturée de chlorure de sodium puis séché sur sulfate de sodium anhydre. Après filtration et évaporation des solvants, on obtient un résidu (720 mg) qui est purifié par filtration sur une colonne de gel de silice en éluant avec un mélange éther éthylique-chlorure de méthylène (1-99 en volumes). On obtient ainsi 413 mg de t.butoxycarbonylamino-3 (éthoxy-1 éthoxy)-2 phényl-3 propionate de méthyle (2R,3S) pur sous forme d'un mélange équimoléculaire des 2 épimères.

Le rendement global est de 55 % à partir du (+)-azido-3 hydroxy-2 phényl-3 propionate de méthyle.

Les caractéristiques sont les suivantes :
- Pouvoir rotatoire : [α]²⁴_{D} = +6,0° (c = 1,52 ; chloroforme).
- Point de fusion : 105-109°C.
- Spectre infra-rouge (film) : 3350,3000, 2950, 2900, 2875, 1722, 1665, 1515, 1420, 1380, 1355, 1320, 1305, 1280, 1250, 1240, 1160, 1130, 1100, 1070, 1035, 1020, 995, 945, 890, 860, 840, 760, 745 et 698 cm⁻¹.
- Spectre de résonance magnétique nucléaire du proton (300 MHz, CDCl₃, déplacements chimiques en ppm, constantes de couplage J en Hz) : 0,79 et 0,96 (2t, J = 7,0, 3H) ; 1,08 et 1,16 (2d, J = 5,4, 3H) ; 1,41 (s large, 9H) ; 2,69-2,75 et 3,14-3,34 (m, 2H) ; 3,757 et 3,762 (2s, 3H) ; 4,34 et 4,46 (2s larges, 1H) ; 4,39 et 4,73 (2q, J= 5,4, 1H) ; 5,22 et 5,54 (2s larges, 1H) ; 7,23-7,33 (m, 5H aromatiques).
- Spectre de masse (i.c., NH₃ + isobutane) : m/e = 385 (MH + NH₃)⁺, 368 (MH)⁺, 339, 332, 296, 283, 257, 240, 222, 206.
- Analyse élémentaire : C % calculé 62,10 trouvé 62,01

H % calculé 7,96 trouvé 7,97

Le (+)-azido-3 hydroxy-2 phényl-3 propionate de méthyle peut être préparé selon l'une des méthodes suivantes :
a) Dans un ballon de 5 cm3, muni d'une agitation magnétique et surmonté d'un réfrigérant, on introduit, sous atmosphère d'argon 84 mg de phényl-3 oxiranecarboxylate de méthyle (2R,3R) (0,472 mmole), 65,1 mg d'azoture de triméthylsilyle (0,57 mmole) et 2-3 mg de chlorure de zinc. On chauffe le mélange réactionnel pendant 20 heures à 72°C. On ajoute 0,47 cm3 d'une solution préparée à partir de 2 cm3 de tétrahydrofuranne, 0,2 cm3 d'acide acétique et deux gouttes d'acide chlorhydrique concentré. L'hydrolyse est terminée après une heure de réaction à une température voisine de 20°C. Au mélange réactionnel, on ajoute 20 cm3 de dichlorométhane et 3 cm3 d'eau. Les deux phases sont séparées par décantation.
   La phase aqueuse est extraite deux fois par 5 cm3 de dichlorométhane. Les phases organiques réunies sont lavées deux fois avec 5 cm3 d'une solution aqueuse saturée de bicarbonate de sodium, trois fois avec 5 cm3 d'eau et 1 fois avec 5 cm3 d'une solution aqueuse saturée de chlorure de sodium puis sont séchées sur sulfate de sodium anhydre. Après filtration et évaporation des solvants sous pression réduite, on obtient un résidu (97 mg) qui est purifié par filtration sur une colonne de gel de silice en éluant avec un mélange éther-hexane (2-8 en volumes). On obtient ainsi, avec un rendement de 87 %, 91 mg de (+)-azido-3 hydroxy-2 phényl-3 propionate de méthyle.
b) Dans un ballon de 50 cm3, muni d'une agitation magnétique et surmonté d'un réfrigérant, on introduit successivement, sous atmosphère d'argon, 430 mg de phényl-3 oxiranecarboxylate de méthyle (2R,3R) (2,4 mmoles), 12 cm3 d'un mélange méthanol-eau (8-1 en volumes) et 800 mg d'azoture de sodium (12,3 mmoles) puis 2 cm3 de formiate de méthyle (3,3 mmoles). On chauffe le mélange réactionnel à 50°C puis on agite pendant 24 heures. Après refroidissement à une température voisine de 20°C, on dilue le mélange réactionnel par addition de 50 cm3 d'éther puis on ajoute 5 cm3 d'eau. Les deux phases sont séparées par décantation. La phase aqueuse est extraite deux fois avec 10 cm3 d'éther éthylique. Les phases organiques réunies sont lavées avec 5 cm3 d'une solution aqueuse saturée de chlorure de sodium et ensuite séchées sur sulfate de sodium anhydre. Après filtration et concentration sous pression réduite, on obtient un résidu qui est purifié par filtration sur gel de silice en éluant avec un mélange acétate d'éthyle-hexane (2-8 en volumes). On obtient, avec un rendement de 90 %, 475 mg de (+)-azido-3 hydroxy-2 phényl-3 propionate de méthyle pur.

Le produit obtenu présente des caractéristiques (spectre infra-rouge, spectre de résonance magnétique nucléaire du proton) identiques à celles qui ont été décrites par J-N. DENIS et coll., J. Org. Chem., 51, 46-50 (1986).
Point de fusion = 56-57°C (pentane) ; [α]²⁴_{D} = +142° (c = 1,1; chloroforme).

### EXEMPLE 2

Dans un ballon de 50 cm3, muni d'une agitation magnétique, on introduit successivement 146,8 mg de t.butoxycarbonylamino-3 (éthoxy-1 éthoxy)-2 phényle-3 propionate de méthyle (2R,3S) (0,4 mmole), 14 cm3 de méthanol distillé, 7 cm3 d'eau et 166 mg de carbonate de potassium solide (1,2 mmole). On agite pendant 40 heures à une température voisine de 20°C puis on évapore le méthanol sous pression réduite. La phase aqueuse basique résiduelle est lavée plusieurs fois à l'éther puis acidifiée par une solution aqueuse d'acide chlorhydrique à 2,5 % (poids/volume) en présence de chlorure de méthylène et ensuite extraite quatre fois par 20 cm3 de chlorure de méthylène. Les phases organiques réunies sont lavées 4 fois avec 5 cm3 d'eau puis 1 fois avec 5 cm3 d'une solution saturée de chlorure de sodium et ensuite séchées sur sulfate de magnésium anhydre. Après filtration et évaporation des solvants sous pression réduite, on obtient, avec un rendement de 76 %, 108 mg d'acide t.butoxycarbonylamino-3 (éthoxy-1 éthoxy)-2 phényl-3 propionique (2R,3S) pur.

Les caractéristiques sont les suivantes :
- Produit déliquescent.
- Pouvoir rotatoire : [α]²⁴_{D} = +17° (c = 1,28 ; chloroforme).
- Spectre infra-rouge (film) : principales bandes d'absorption caractéristiques à : 3700-2200, 3060, 2980, 2930, 1720, 1660, 1602, 1590, 1500, 1450, 1400, 1370, 1280, 1250, 1170, 1080, 1050, 1030, 955, 930, 890 et 700 cm⁻¹.
- Spectre de résonance magnétique nucléaire du proton (300 MHz, CDCl₃, déplacements chimiques en ppm, constantes de couplage J en Hz) : 0,81 et 1,04 (2t, J = 7,3H) ; 1,18 et 1,20 (2d, J = 5,4, 3H) ; 1,42 (s, 9H) ; 2,60-2,88 et 3,15-3,52 (m, 2H) ; 4,35-4,50 et 4,65-4,80 (m, 2H) ; 5,29 (s large, 1H) ; 5,72 (s large, 1H) ; 7,13-7,38 (m, 5H aromatiques) ; 8,52 (s large, 1H).

### EXEMPLE 3

Un mélange de 1,51 g (6,83 mmoles) de (+)-azido-3 hydroxy-2 phényl-3 propionate de méthyle-(2R,3S), 1,93 g (13,7 mmoles) de chlorure de benzoyle, 2,07 g (20,4 mmoles) de triéthylamine et 30,2 mg (0,25 mmole) de diméthylamino-4 pyridine dans 25 cm3 d'acétate d'éthyle est agité sous atmosphère d'argon à 20°C pendant 4 heures puis on ajoute 1,4 cm3 de méthanol. On agite encore pendant 3 heures puis on ajoute 152 mg de palladium sur charbon à 10 % (p/p) et on place sous atmosphère d'hydrogène. Le mélange est agité pendant 68 heures. On remplace l'hydrogène par de l'argon, on dilue le mélange réactionnel dans 100 cm3 de chlorure de méthylène et on élimine le palladium sur charbon par filtration sous vide sur célite. On lave les solides 3 fois avec 20 à 30 cm3 de chlorure de méthylène puis on élimine les solvants sous vide. On obtient un produit brut qui est purifié par chromatographie sur gel de silice en éluant avec un mélange éther-dichlorométhane (5-95 en volumes). On obtient ainsi 1,88 g d'ester méthylique de la (-)-N-benzoyl-phényl-3 isosérine-(2R,3S) dont les caractéristiques sont les suivantes :
- Point de fusion : 184-185°C (chlorure de méthylène-cyclohexane)
- Pouvoir rotatoire : [α]²⁴_{D} = -48° (c = 1 ; méthanol)

Le rendement est de 92 %.

Le (+)-azido-3 hydroxy-2 phényl-3 propionate de méthyle(2R,3S) peut être préparé de la manière suivante :

On traite 1,35 g (7,58 mmoles) de (+)-phényl-3 oxirane carboxylate de méthyle-(2R,3R) en solution dans 40 cm3 d'un mélange méthanol-eau (8-1 en volumes) par 6,3 cm3 de formiate de méthyle et 2,46 g (37,8 mmoles) d'azoture de sodium. On agite pendant 46 heures à 50°C sous atmosphère d'argon. Le produit obtenu est extrait à l'éther dans les conditions habituelles. Le produit brut obtenu est purifié par chromatographie sur gel de silice en éluant par de l'hexane contenant 10 % d'acétate d'éthyle. On obtient ainsi 1,59 g de (+)-azido-3 hydroxy-2 phényl-3 propionate de méthyl-(2R,3S) dont les carastéristiques sont les suivantes :
- Point de fusion : 56-57°C (pentane)
- Pouvoir rotatoire : [α]²⁴_{D} = +142° (c = 1,1 ; chloroforme)

Le rendement est de 95 %.

### EXEMPLE 4

On agite pendant 10 minutes sous atmosphère d'hydrogène une suspension de 148 mg de palladium sur charbon à 10 % (p/p) dans 3 cm3 d'acétate d'éthyle à 20°C puis on ajoute une solution de 1,75 g (8,02 mmoles) de dicarbonate de di-tert-butyle et de 1,48 g (6,70 mmoles) de (+)-azido-3 hydroxy-2 phényl-3 propionate de méthyle- (2R,3S) dans 12 cm3 d'acétate d'éthyle.

On agite le mélange pendant 56 heures. On remplace l'hydrogène par de l'argon, on dilue le mélange dans 100 cm3 d'acétate d'éthyle et on élimine le palladium sur charbon par filtration sous vide sur célite. On lave les solides 3 fois avec 30 à 40 cm3 d'acétate d'éthyle et on élimine le solvant sous vide. On obtient un produit brut qui est purifié par chromatographie sur gel de silice en éluant avec un mélange éther-dichlorométhane (5-95 en volumes). On obtient ainsi 1,81 g de l'ester méthylique de la (-)-N-(tert-butoxycarbonyl) phényl-3 isosérine-(2R,3S) dont les caractéristiques sont les suivantes :
- Point de fusion : 130,5-131,5°C (chlorure de méthylène-cyclohexane)
- Pouvoir rotatoire : [α]²⁴_{D} = -7° (c = 1,2 ; choroforme)
- Spectre infra-rouge : principales bandes d'absorption caractéristiques à 3500, 3380, 3110, 3060, 3000, 2975, 2930, 1735, 1690, 1518, 1500, 1442, 1390, 1360, 1300, 1250, 1170, 1100, 1050, 1030, 980, 940, 930, 900 et 705 cm⁻¹
- Spectre de résonance magnétique nucléaire du proton (déplacements chimiques en ppm, constantes de couplage J en Hz) : 1,42 (s large, 9H); 3,11 (s large, 1H) ; 3,84 (s, 3H) ; 4,47 (s large, 1H) ; 5,21 (d déformé, J = 9,4, 1H) ; 5,36 (d déformé, J = 8,5, 1H) ; 7,26-7,37 (m, 5H).
- Spectre de masse (i.c., NH₃ + isobutane) : m/e = 313 (MH + NH₃)⁺, 296 (MH⁺), 257, 240, 206, 196.
- Analyse élémentaire : C % calculé 61,00 trouvé 60,85

H % calculé 7,17 trouvé 7,17

Le rendement est de 92 %.

## Revendications

1. Procédé de préparation énantiosélective de dérivés de la phénylisosérine de formule générale : 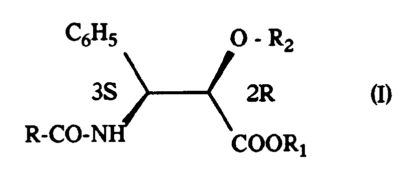 dans laquelle R représente un radical phényle ou tert.butoxy, R₁ représente un atome d'hydrogène ou un radical alkyle contenant 1 à 4 atomes de carbone et R₂ représente un atome d'hydrogène ou un groupement protecteur de la fonction alcool, caractérisé en ce que :
- pour préparer un produit de formule générale (I) dans laquelle R₂ représente un groupement protecteur de la fonction hydroxy, on protège selon des méthodes connues la fonction alcool d'un hydroxy azoture de formule générale : 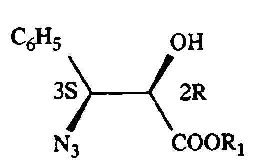 dans laquelle R₁ représente un radical alkyle contenant 1 à 4 atomes de carbone, qui est obtenu par action d'un azoture choisi parmi les azotures alcalins ou l'azoture de triméthylsilyle sur un dérivé de l'oxirane de formule générale : 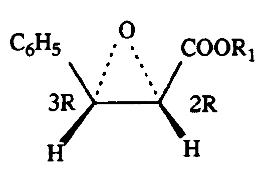 dans laquelle R₁ est défini comme précédemment, pour obtenir un produit de formule générale : 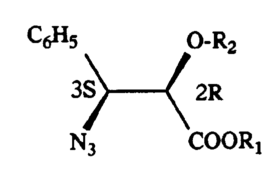 dans laquelle R₁ et R₂ sont définis comme précédemment, que l'on réduit pour obtenir un produit de formule générale : 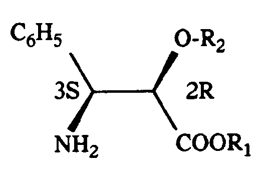 dans laquelle R₁ et R₂ sont définis comme précédemment, que l'on traite éventuellement in situ par un réactif permettant d'introduire le groupement benzoyle ou t.butoxycarbonyle pour obtenir le dérivé de la phénylisosérine de formule générale (I) dans laquelle R₁ représente un radical alkyle contenant 1 à 4 atomes de carbone, que l'on peut saponifier pour obtenir un produit de formule générale (I) dans laquelle R₁ représente un atome d'hydrogène et R₂ représente un groupement protecteur de la fonction hydroxy,
- pour préparer un produit de formule générale (I) dans laquelle R₂ représente un atome d'hydrogène ou un groupement protecteur de la fonction hydroxy, on acyle et hydrogène simultanément ou successivement, sans isoler les produits intermédiaires de réaction, l'hydroxy-azoture de formule générale : 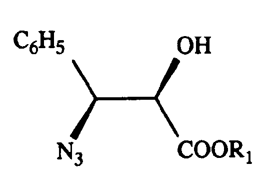 dans laquelle R₁ représente un radical alkyle contenant 1 à 4 atomes de carbone, pour obtenir un produit de formule générale (I) dans laquelle R₁ représente un radical alkyle contenant 1 à 4 atomes de carbone et R₂ représente un atome d'hydrogène, que l'on peut soit saponifier pour obtenir un produit de formule générale (I) dans laquelle R₁ et R₂ représente chacun un atome d'hydrogène, soit protéger puis saponifier pour obtenir le produit de formule générale (I) dans laquelle R₁ représente un atome d'hydrogène et R₂ représente un groupement protecteur de la fonction hydroxy.

2. Procédé selon la revendication 1 pour la préparation d'un produit de formule générale (I) dans laquelle R₁ représente un atome d'hydrogène ou un radical alkyle contenant 1 à 4 atomes de carbone et R₂ représente un atome d'hydrogène ou un groupement protecteur de la fonction alcool choisi parmi les radicaux méthoxyméthyle, éthoxy-1 éthyle, benzyloxyméthyle, (β-triméthylsilyléthoxy) méthyle, tétrahydropyrannyle et trichloro-2,2,2 éthoxycarbonyle.

3. Procédé selon l'une des revendications 1 ou 2 pour la préparation d'un produit de formule générale (I) dans laquelle R₁ représente un atome d'hydrogène ou un radical alkyle contenant 1 à 4 atomes de carbone et R₂ représente un groupement protecteur de la fonction hydroxy, caractérisé en ce que la réduction de l'hydroxy azoture est effectuée au moyen d'hydrogène en présence d'un catalyseur d'hydrogénation en opérant dans un alcool inférieur.

4. Procédé selon la revendication 3 caractérisé en ce que le catalyseur est le palladium sur charbon et le solvant est le méthanol.

5. Procédé selon l'une des revendications 1 ou 2 pour la préparation d'un produit de formule générale (I) dans laquelle R₁ représente un atome d'hydrogène ou un radical alkyle contenant 1 à 4 atomes de carbone et R₂ représente un groupement protecteur de la fonction hydroxy caractérisé en ce que, pour introduire le groupement benzoyle ou t.butoxycarbonyle, on utilise le chlorure de benzoyle ou le dicarbonate de di-tert.butyle en opérant dans un solvant organique inerte en présence d'une base minérale ou organique.

6. Procédé selon la revendication 5 caractérisé en ce que le solvant est choisi parmi les hydrocarbures halogénés tels que le chlorure de méthylène et la base est la triéthylamine.

7. Procédé selon l'une des revendications 1 ou 2 pour la préparation d'un produit de formule générale (I) dans laquelle R₁ représente un radical alkyle contenant 1 à 4 atomes de carbone et R₂ représente un atome d'hydrogène caractérisé en ce que l'on fait réagir l'anhydride benzoïque ou le dicarbonate de di-tert.butyle en présence d'hydrogène et d'un catalyseur d'hydrogénation en opérant dans un solvant organique inerte à une température comprise entre 0 et 40°C.

8. Procédé selon la revendication 7 caractérisé en ce que le catalyseur est du palladium sur charbon.

9. Procédé selon la revendication 7 caractérisé en ce que le solvant est choisi parmi l'acétate de méthyle et l'acétate d'éthyle.

10. Procédé selon l'une des revendications 1 ou 2 pour la préparation d'un produit de formule générale (I) dans laquelle R représente un radical phényle, R₁ représente un radical alkyle contenant 1 à 4 atomes de carbone et R₂ représente un atome d'hydrogène caractérisé en ce que l'on fait réagir le chlorure de benzoyle en présence d'une base organique et d'un agent d'activation sur l'hydroxy-azoture puis, après addition de méthanol, place le mélange réactionnel sous atmosphère d'hydrogène en présence d'un catalyseur d'hydrogénation en opérant dans un solvant organique inerte à une température comprise entre 0 et 40°C.

11. Procédé selon la revendication 10 caractérisé en ce que la base organique est la triéthylamine.

12. Procédé selon la revendication 10 caractérisé en ce que l'agent d'activation est la diméthylamino-4 pyridine.

13. Procédé selon la revendication 10 caractérisé en ce que le catalyseur d'hydrogénation est du palladium sur charbon.

14. Procédé selon la revendication 10 caractérisé en ce que le solvant est choisi parmi l'acétate de méthyle et l'acétate d'éthyle.

15. Procédé selon l'une des revendications 1 ou 2 pour la préparation d'un produit de formule générale (I) dans laquelle R₁ représente un atome d'hydrogène caractérisé en ce que l'on traite un produit selon l'une des revendications 1 ou 2 dans laquelle R₁ représente un radical alkyle contenant 1 à 4 atomes de carbone par une base minérale choisie parmi les hydroxydes, les carbonates et les bicarbonates de métaux alcalins en milieu hydroalcoolique à une température comprise entre 0 et 40°C.

16. Procédé de préparation d'un dérivé de la baccatine III ou de la désacétyl-10 baccatine III de formule générale : 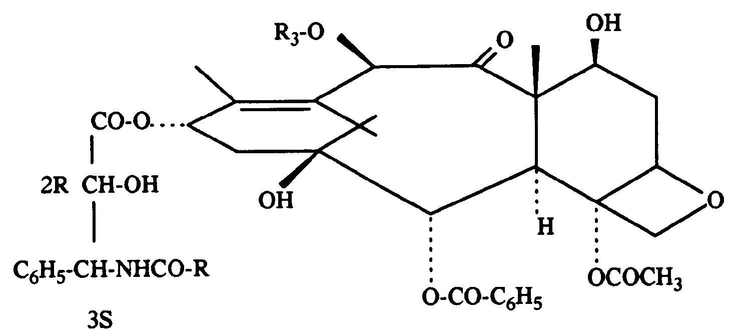 dans laquelle R représente un radical phényle ou tert.butoxy et R₃ représente un atome d'hydrogène ou un radical acétyle caractérisé en ce que l'on fait agir un azoture choisi parmi les azotures alcalins ou l'azoture de triméthylsilyle sur un dérivé de l'oxirane de formule générale : 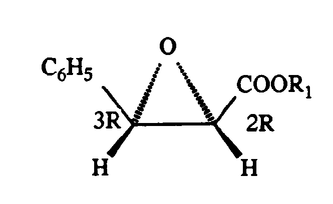 dans laquelle R₁ représente un atome d'hydrogène ou un radical alcoyle contenant 1 à 4 atomes de carbone, pour obtenir un produit de formule générale : 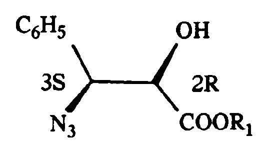 sur lequel on fait réagir simultanément ou successivement, après avoir éventuellement protégé la fonction hydroxy, l'hydrogène et un réactif permettant d'introduire un radical benzoyle ou t.butoxycarbonyle, sans isoler les produits intermédiaires de réaction, pour obtenir, après protection de la fonction hydroxy, un produit de formule générale : 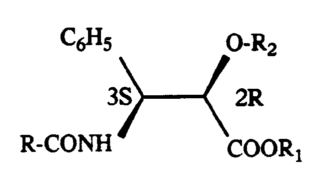 dans laquelle R₁ est défini comme précédemment et R₂ représente un groupement protecteur de la fonction hydroxy, que l'on fait réagir, après saponification éventuelle, sur un produit de formule générale : 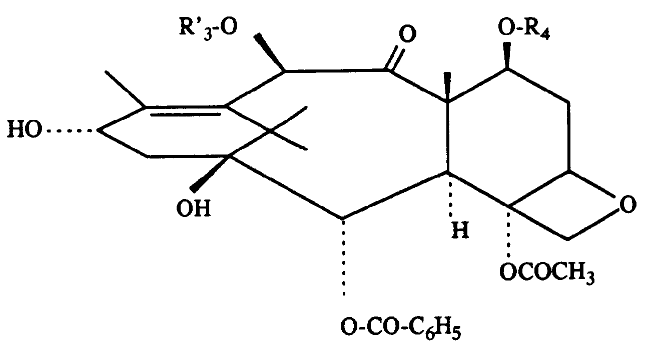 dans laquelle R'₃ représente un radical acétyle ou un groupement protecteur de la fonction hydroxy et R₄ représente un groupement protecteur de la fonction hydroxy, pour obtenir un produit de formule générale : 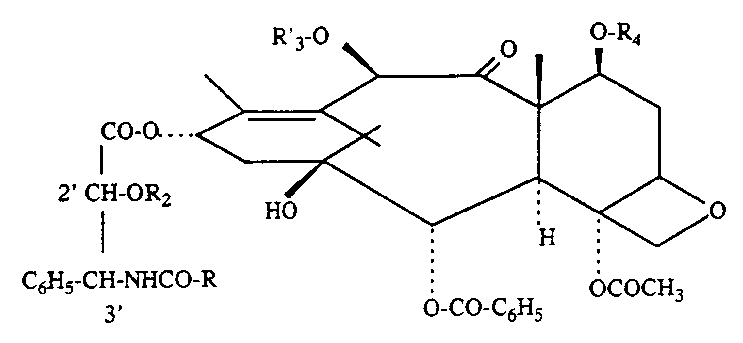 dont on remplace les groupements protecteurs R₂, R'₃ et R₄ par des atomes d'hydrogène et isole le produit ainsi obtenu.

17. Procédé selon la revendication 16 caractérisé en ce que les groupements protecteurs représentés par R'₃ et R₄ sont choisis parmi les groupements trichloro-2,2,2 éthoxycarbonyle ou trialkylsilyle dont chaque partie alkyle contient 1 à 3 atomes de carbone.

## Patentansprüche

1. Verfahren zur enantioselektiven Herstellung von Phenylisoserin-Derivaten der allgemeinen Formel 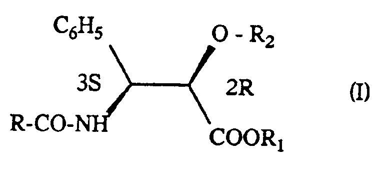 worin R für einen Phenyl- oder tert.-Butoxyrest steht, R₁ ein Wasserstoffatom oder einen Alkylrest mit 1 bis 4 Kohlenstoffatomen bedeutet, und R₂ ein Wasserstoffatom oder eine Schutzgruppe für die Alkoholfunktion wiedergibt, dadurch gekennzeichnet, daß man
- zur Herstellung eines Produkts der allgemeinen Formel (I), worin R₂ eine Schutzgruppe für die Hydroxyfunktion bedeutet, nach bekannten Methoden die Alkoholfunktion eines Hydroxyazids der allgemeinen Formel 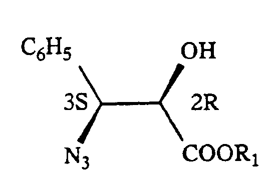 worin R₁ für einen Alkylrest mit 1 bis 4 Kohlenstoffatomen steht, schützt, welches erhalten wird durch Umsetzung eines Azids, ausgewählt unter den Alkaliaziden oder dem Trimethylsilylazid mit einem Oxiran der allgemeinen Formel 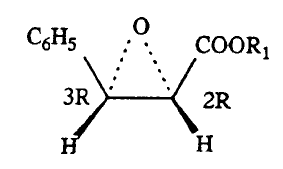 worin R₁ wie vorstehend definiert ist, um zu einem Produkt der allgemeinen Formel 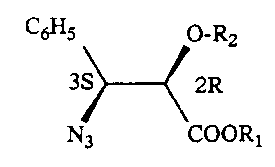 zu gelangen, worin R₁ und R₂ wie vorstehend definiert sind, welches man reduziert, um ein Produkt der allgemeinen Formel 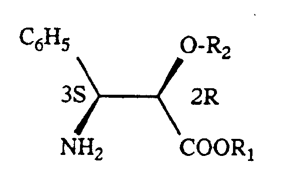 zu erhalten, in dem R₁ und R₂ wie vorstehend definiert sind, das man gegebenenfalls in situ mit einem Reagens behandelt, welches die Einführung der Benzoyl- oder tert.- Butoxycarbonylgruppe ermöglicht, um zu dem Phenylisoserin derivat der allgemeinen Formel (I) zu gelangen, worin R₁ einen Alkylrest mit 1 bis 4 Kohlenstoffatomen bedeutet, welches man verseifen kann, um ein Produkt der allgemeinen Formel (I) zu erhalten, worin R₁ für ein Wasserstoffatom steht, und R₂ eine Schutzgruppe für die Hydroxyfunktion darstellt,
- zur Herstellung eines Produkts der allgemeinen Formel (I), worin R₂ ein Wasserstoffatom oder eine Schutzgruppe für die Hydroxyfunktion bedeutet, gleichzeitig oder nacheinander ohne Isolierung der Reaktionszwischenprodukte das Hydroxyazid der allgemeinen Formel 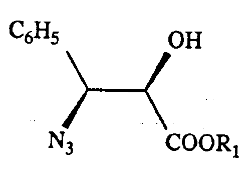
worin R₁ für einen Alkylrest mit 1 bis 4 Kohlenstoffatomen steht, acyliert und hydriert, um zu einem Produkt der allgemeinen Formel (I) zu gelangen, worin R₁ einen Alkylrest mit 1 bis 4 Kohlenstoffatomen bedeutet, und R₂ für ein Wasserstoffatom steht, welches man entweder verseifen kann, um zu einem Produkt der allgemeinen Formel (I) zu gelangen, worin R₁ und R₂ jeweils ein Wasserstoffatom bedeuten, oder mit einer Schutzgruppe versehen und danach verseifen kann, um zu dem Produkt der allgemeinen Formel (I) zu gelangen, worin R₁ ein Wasserstoffatom bedeutet, und R₂ eine Schutzgruppe für die Hydroxyfunktion wiedergibt.

2. Verfahren gemäß Anspruch 1 zur Herstellung eines Produkts der allgemeinen Formel (I), worin R₁ für ein Wasserstoffatom oder einen Alkylrest mit 1 bis 4 Kohlenstoffatomen steht, und R₂ ein Wasserstoffatom oder eine Schutzgruppe für die Alkoholfunktion, ausgewählt unter den Methoxymethyl-, 1-Ethoxyethyl-, Benzyloxymethyl-, (β-Trimethylsilylethoxy)-methyl-, T etra-hydropyranyl- und 2,2,2-Trichlorethoxycarbonylresten, bedeutet.

3. Verfahren gemäß einem der Ansprüche 1 oder 2 zur Herstellung eines Produkts der allgemeinen Formel (I), worin R₁ ein Wasserstoffatom oder einen Alkylrest mit 1 bis 4 Kohlenstoffatomen bedeutet, und R₂ für eine Schutzgruppe der Hydroxyfunktion steht, dadurch gekennzeichnet, daß die Reduktion des Hydroxyazids mit Hilfe von Wasserstoff in Gegenwart eines Hydrierungskatalysators durchgeführt wird, wobei man in einem niedrigen Alkohol arbeitet.

4. Verfahren gemäß Anspruch 3, dadurch gekennzeichnet, daß der Katalysator Palladium auf Kohle und das Lösungsmittel Methanol ist.

5. Verfahren gemäß einem der Ansprüche 1 oder 2 zur Herstellung eines Produkts der allgemeinen Formel (I), worin R₁ für ein Wasserstoffatom oder einen Alkylrest mit 1 bis 4 Kohlenstoffatomen steht, und R₂ eine Schutzgruppe für die Hydroxyfunktion wiedergibt, dadurch gekennzeichnet, daß man zur Einführung der Benzoyl- oder tert.-Butoxycarbonylgruppe Benzoylchlorid oder Di-tert.-butyldicarbonat verwendet, wobei man in einem inerten organischen Lösungsmittel in Gegenwart einer mineralischen oder organischen Base arbeitet.

6. Verfahren gemäß Anspruch 5, dadurch gekennzeichnet, daß das Lösungsmittel unter den halogenierten Kohlenwasserstoffen, wie Methylenchlorid, ausgewählt wird, und die Base Triethylamin ist.

7. Verfahren gemäß einem der Ansprüche 1 oder 2 zur Herstellung eines Produkts der allgemeinen Formel (I), worin R₁ für einen Alkylrest mit 1 bis 4 Kohlenstoffatomen steht, und R₂ ein Wasserstoffatom bedeutet, dadurch gekennzeichnet, daß man Benzoesäureanhydrid oder Di-tert.-butyldicarbonat in Gegenwart von Wasserstoff und einem Hydrierungskatalysator umsetzt, wobei man in einem inerten organischen Lösungsmittel bei einer Temperatur zwischen 0 und 40°C arbeitet.

8. Verfahren gemäß Anspruch 7, dadurch gekennzeichnet, daß der Katalysator Palladium auf Kohle ist.

9. Verfahren gemäß Anspruch 7, dadurch gekennzeichnet, daß das Lösungsmittel unter Methylacetat und Ethylacetat ausgewählt wird.

10. Verfahren gemäß einem der Ansprüche 1 oder 2 zur Herstellung eines Produkts der allgemeinen Formel (I), worin R für einen Phenylrest steht, R₁ einen Alkylrest mit 1 bis 4 Kohlenstoffatomen bedeutet, und R₂ ein Wasserstoffatom bedeutet, dadurch gekennzeichnet, daß man Benzoylchlorid in Gegenwart einer organischen Base und eines Aktivierungsmittels mit dem Hydroxyazid umsetzt, und dann nach Zusatz von Methanol das Reaktionsgemisch unter Wasserstoffatmosphäre in Gegenwart eines Hydrierungskatalysators bringt, wobei man in einem inerten organischen Lösungsmittel bei einer Temperatur zwischen 0 und 40°C arbeitet.

11. Verfahren gemäß Anspruch 10, dadurch gekennzeichnet, daß die organische Base Triethylamin ist.

12. Verfahren gemäß Anspruch 10, dadurch gekennzeichnet, daß das Aktivierungsmittel 4-Dimethylaminopyrridin ist.

13. Verfahren gemäß Anspruch 10, dadurch gekennzeichnet, daß der Hydrierungskatalysator Palladium auf Kohle ist.

14. Verfahren gemäß Anspruch 10, dadurch gekennzeichnet, daß das Lösungsmittel unter Methylacetat und Ethylacetat ausgewählt wird.

15. Verfahren gemäß einem der Ansprüche 1 oder 2 zur Herstellung eines Produkts der allgemeinen Formel (I), worin R₁ für ein Wasserstoffatom steht, dadurch gekennzeichnet, daß man ein Produkt gemäß einem der Ansprüche 1 oder 2, worin R₁ für einen Alkylrest mit 1 bis 4 Kohlenstoffatomen steht, mit einer mineralischen Base, ausgewählt unter den Alkalimetallhydroxiden, -carbonaten und -bicarbonaten, in wäßrig-alkoholischem Milieu bei einer Temperatur zwischen 0 und 40°C behandelt.

16. Verfahren zur Herstellung eines Derivats des Baccatin-III oder des Desacetyl-10-baccatin-III der allgemeinen Formel 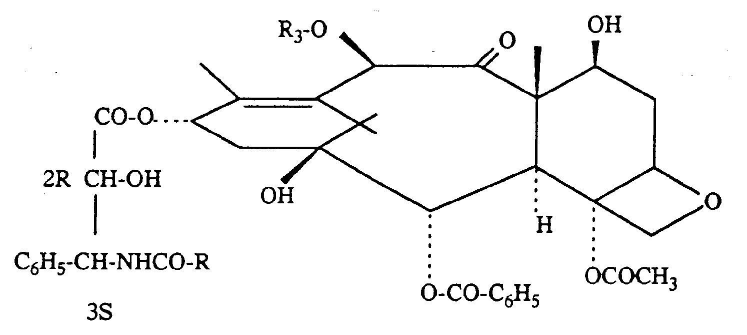 worin R für einen Phenyl- oder tert.-Butoxyrest steht, und R₃ ein Wasserstoffatom oder einen Acetylrest bedeutet, dadurch gekennzeichnet, daß man ein Azid, ausgewählt unter den Alkaliaziden oder Trimethylsilylazid, mit einem Oxiranderivat der allgemeinen Formel 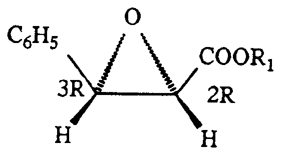 worin R₁ für ein Wasserstoffatom oder einen Alkylrest mit 1 bis 4 Kohlenstoffatomen steht, umsetzt, um zu einem Produkt der allgemeinen Formel 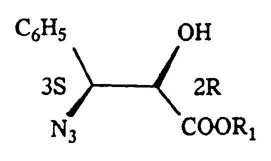 zu gelangen, mit dem man gleichzeitig oder nacheinander, nachdem man gegebenenfalls die Hydroxyfunktion geschützt hat, Wasserstoff und ein Reagens, welches die Einführung eines Benzoyl- oder tert.-Butoxycarbonylrestes ermöglicht, ohne Isolierung der Reaktionszwischenprodukte reagieren läßt, um nach Schutz der Hydroxyfunktion ein Produkt der allgemeinen Formel 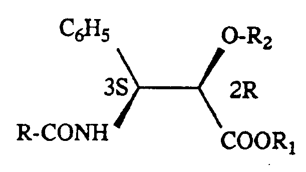 zu erhalten, worin R₁ wie vorstehend definiert ist, und R₂ eine Schutzgruppe für die Hydroxyfunktion darstellt, welches man nach etwaiger Verseifung mit einem Produkt der allgemeinen Formel 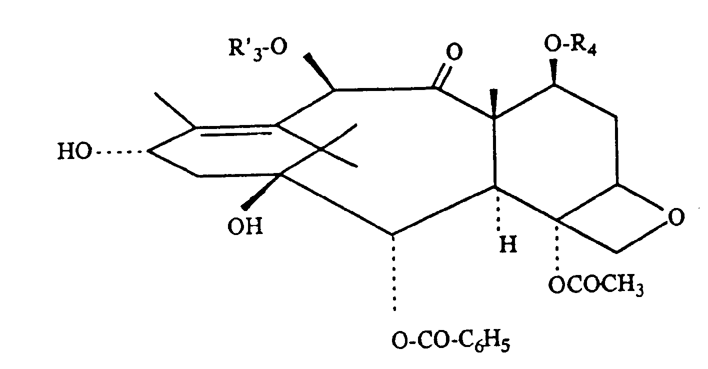 umsetzt, worin R'₃ einen Acetylrest oder eine Schutzgruppe für die Hydroxyfunktion bedeutet, und R₄ eine Schutzgruppe für die Hydroxyfunktion darstellt, um zu einem Produkt der allgemeinen Formel 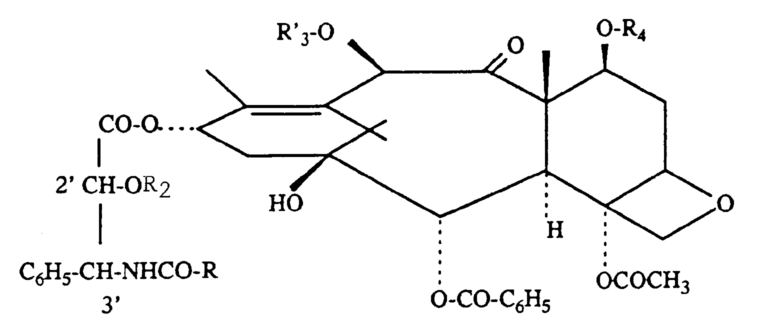 zu gelangen, dessen Schutzgruppen R₂, R'₃ und R₄ man durch Wasserstoffatome ersetzt und das so erhaltene Produkt isoliert.

17. Verfahren gemäß Anspruch 16, dadurch gekennzeichnet, daß die durch R'₃ und R₄ dargestellten Schutzgruppen unter den 2,2,2-Trichlorethoxycarbonyl- oder Trialkylsilylgruppen, deren Alkylteil 1 bis 3 Kohlenstoffatome aufweist, ausgewählt werden.

## Claims

1. Process for the enantioselective preparation of phenylisoserine derivatives of general formula: 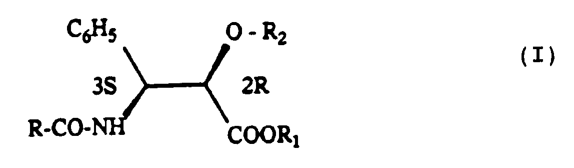 in which R represents a phenyl or tert-butoxy radical, R₁ represents a hydrogen atom or an alkyl radical containing 1 to 4 carbon atoms and R₂ represents a hydrogen atom or a protective group for the alcohol functional group, characterised in that:
- in order to prepare a product of general formula (I) in which R₂ represents a protective group for the hydroxyl functional group, the alcohol functional group of a hydroxyazide of general formula: 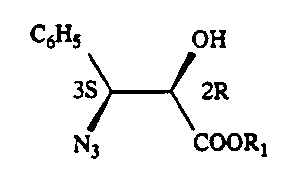 in which R₁ represents an alkyl radical containing 1 to 4 carbon atoms, which is obtained by the action of an azide chosen from alkali metal azides or trimethylsilyl azide on an oxirane derivative of general formula: 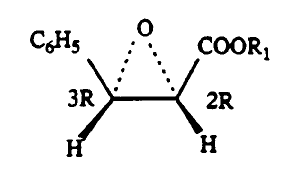 in which R₁ is defined as above, is protected by known methods in order to obtain a product of general formula: 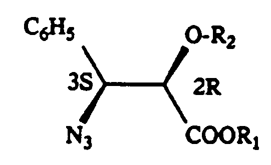 in which R₁ and R₂ are defined as above, which product is reduced in order to obtain a product of general formula: 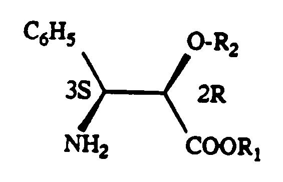 in which R₁ and R₂ are defined as above, which product is treated, optionally in situ, with a reactant allowing a benzoyl or t-butoxycarbonyl group to be introduced in order to obtain the phenylisoserine derivative of general formula (I) in which R₁ represents an alkyl radical containing 1 to 4 carbon atoms, which derivative may be saponified in order to obtain a product of general formula (I) in which R₁ represents a hydrogen atom and R₂ represents a protective group for the hydroxyl functional group, or
- in order to prepare a product of general formula (I) in which R₂ represents a hydrogen atom or a protective group for the hydroxyl functional group, the hydroxyazide of general formula: 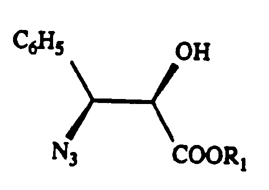
in which R₁ represents an alkyl radical containing 1 to 4 carbon atoms is acylated and hydrogenated, simultaneously or successively, without isolating the intermediate reaction products, in order to obtain a product of general formula (I) in which R₁ represents an alkyl radical containing 1 to 4 carbon atoms and R₂ represents a hydrogen atom, which product may either be saponified in order to obtain a product of general formula (I), in which R₁ and R₂ each represent a hydrogen atom, or be protected and then saponified in order to obtain the product of general formula (I) in which R₁ represents a hydrogen atom and R₂ represents a protective group for the hydroxyl functional group.

2. Process according to Claim 1 for the preparation of a product of general formula (I) in which R₁ represents a hydrogen atom or an alkyl radical containing 1 to 4 carbon atoms and R₂ represents a hydrogen atom or a protective group for the alcohol functional group, chosen from methoxymethyl, 1-ethoxyethyl, benzyloxymethyl, (β-trimethylsilylethoxy)methyl, tetrahydropyranyl and 2,2,2-trichloroethoxycarbonyl radicals.

3. Process according to either of Claims 1 and 2 for the preparation of a product of general formula (I) in which R₁ represents a hydrogen atom or an alkyl radical containing 1 to 4 carbon atoms and R₂ represents a protective group for the hydroxyl functional group, characterised in that the reduction of the hydroxyazide is carried out by means of hydrogen in the presence of a hydrogenation catalyst, the reaction being carried out in a lower alcohol.

4. Process according to Claim 3, characterised in that the catalyst is palladium on charcoal and the solvent is methanol.

5. Process according to either of Claims 1 and 2 for the preparation of a product of general formula (I) in which R₁ represents a hydrogen atom or an alkyl radical containing 1 to 4 carbon atoms and R₂ represents a protective group for the hydroxyl functional group, characterised in that benzoyl chloride or di-tert-butyl dicarbonate is used in order to introduce the benzoyl or t-butoxycarbonyl group, the reaction being carried out in an inert organic solvent in the presence of an inorganic or organic base.

6. Process according to Claim 5, characterised in that the solvent is chosen from halogenated hydrocarbons, such as methylene chloride, and the base is triethylamine.

7. Process according to either of Claims 1 and 2 for the preparation of a product of general formula (I) in which R₁ represents an alkyl radical containing 1 to 4 carbon atoms and R₂ represents a hydrogen atom, characterised in that benzoic anhydride or di-tert-butyl dicarbonate is reacted in the presence of hydrogen and a hydrogenation catalyst, the reaction being carried out in an inert organic solvent at a temperature of between 0 and 40°C.

8. Process according to Claim 7, characterised in that the catalyst is palladium on charcoal.

9. Process according to Claim 7, characterised in that the solvent is chosen from methyl acetate and ethyl acetate.

10. Process according to either of Claims 1 and 2 for the preparation of a product of general formula (I) in which R represents a phenyl radical, R₁ represents an alkyl radical containing 1 to 4 carbon atoms and R₂ represents a hydrogen atom, characterised in that benzoyl chloride is reacted with the hydroxyazide in the presence of an organic base and of an activating agent and then, after addition of methanol, the reaction mixture is placed under a hydrogen atmosphere in the presence of a hydrogenation catalyst, the reaction being carried out in an inert organic solvent at a temperature of between 0 and 40°C.

11. Process according to Claim 10, characterised in that the organic base is triethylamine.

12. Process according to Claim 10, characterised in that the activating agent is 4-dimethylaminopyridine.

13. Process according to Claim 10, characterised in that the hydrogenation catalyst is palladium on charcoal.

14. Process according to Claim 10, characterised in that the solvent is chosen from methyl acetate and ethyl acetate.

15. Process according to either of Claims 1 and 2 for the preparation of a product of general formula (I) in which R₁ represents a hydrogen atom, characterised in that a product according to either of Claims 1 and 2 in which R₁ represents an alkyl radical containing 1 to 4 carbon atoms is treated with an inorganic base chosen from alkali metal hydroxides, carbonates and bicarbonates in an aqueous-alcoholic medium at a temperature of between 0 and 40°C.

16. Process for the preparation of a baccatin III derivative or 10-deacetyl baccatin III derivative of general formula: 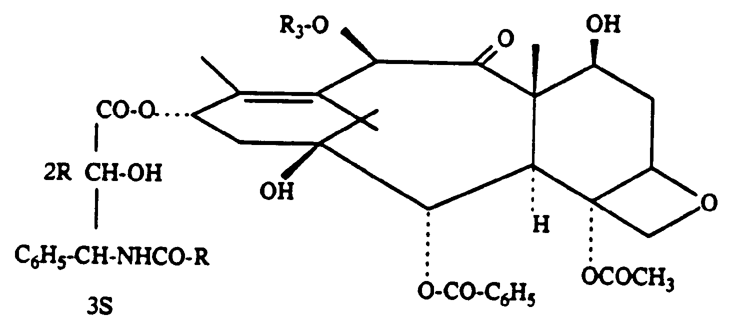 in which R represents a phenyl or tert-butoxy radical and R₃ represents a hydrogen atom or an acetyl radical, characterised in that an azide chosen from alkali metal azides or trimethylsilyl azide is reacted with an oxirane derivative of general formula: 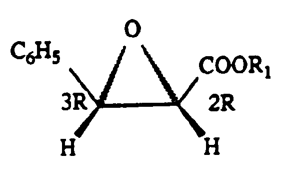 in which R₁ represents a hydrogen atom or an alkyl radical containing 1 to 4 carbon atoms, in order to obtain a product of general formula: 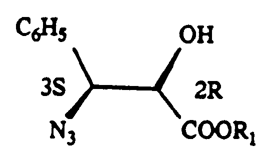 with which, after having protected the hydroxyl functional group if appropriate, hydrogen and a reagent permitting the introduction of a benzoyl or t-butoxy-carbonyl radical are reacted, simultaneously or successively, without isolating the intermediate reaction products, in order to obtain, after protection of the hydroxyl functional group, a product of general formula: 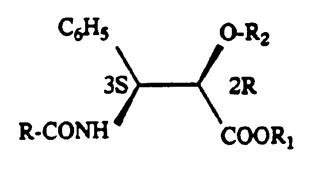 in which R₁ is defined as above and R₂ represents a protective group for the hydroxyl functional group, which product is reacted, after saponification if appropriate, with a product of general formula: 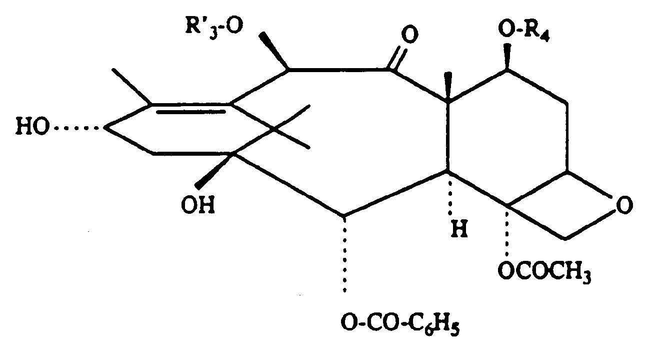 in which R'₃ represents an acetyl radical or a protective group for the hydroxyl functional group and R₄ represents a protective group for the hydroxyl functional group, in order to obtain a product of general formula: 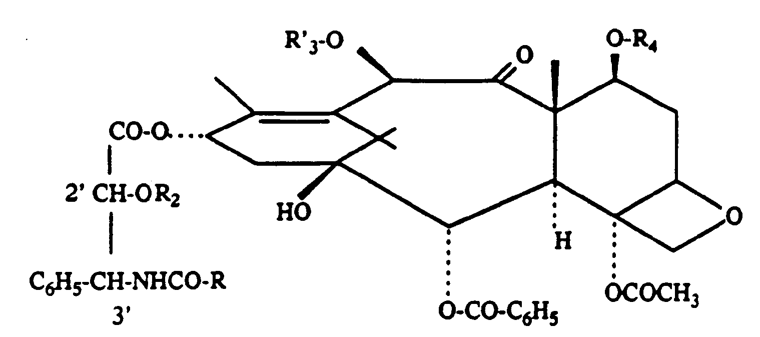 in which product the protective groups R₂, R'₃ and R₄ are replaced by hydrogen atoms and the product thus obtained is isolated.

17. Process according to Claim 16, characterised in that the protective groups represented by R'₃ and R₄ are chosen from 2,2,2-trichloroethoxycarbonyl groups or trialkylsilyl groups in which each alkyl part contains 1 to 3 carbon atoms.
